# EUROPEAN PATENT SPECIFICATION

(11) **EP 1 636 365 B1**
(45) Date of publication and mention of the grant of the patent: **04.08.2010**
(21) Application number: 04736173.8
(22) Date of filing: 04.06.2004
(51) Int. Cl.: C12N 15/12, C12N 15/63, C07K 7/00, C07K 14/435, A61K 38/00, A61P 7/02, A61P 9/10

(54) **CHOLESTEROL BIOSYNTHESIS PATHWAY MODULATORS AND USES THEREOF**
MODULATOREN DES CHOLESTERINBIOSYNTHESEWEGS UND VERWENDUNGEN DAVON
MODULATEURS DE LA VOIE BIOSYNTHESE DE CHOLESTEROL ET LEURS UTILISATIONS

(30) Priority: 05.06.2003 US 476208 P
(43) Date of publication of application: 22.03.2006
(73) Proprietor: NATIONAL UNIVERSITY OF SINGAPORE, Singapore 119260 (SG)
(72) Inventor: Jeyaseelan, Kandiah, Donvale, VIC 3111 (AU); Armugam, Arunmozhiarasi, c/o National University, Singapore 119260 (SG); Chai, Siaw Ching, c/o National University, Singapore 119260 (SG); Ramkishen, Prabhakaran N., Singapore 440029 (SG); Gopalakrishnakone, Ponnampalam, Singapore 596552 (SG); Tan, Kwong Huat, Benny, Singapore 269565 (SG)
(74) Representative: Arends, William Gerrit
(86) International application number: PCT/SG2004/000168
(87) International publication number: WO 2004/108928

(56) References cited:
- EP-A1- 0 671 171
- DATABASE MEDLINE [Online] 16 August 2004 MURTHY R.K. ET AL.: 'Increase in serum free fatty acids, phospholipids and reduction in total cholesterol in acute myocarditis produced by scorpion (Buthus tamulus) venom injection in dogs', XP002994655 Database accession no. (NLM3570321) & INDIAN HEART JOURNAL vol. 38, no. 5, 1986, pages 369 - 372
- DATABASE CA [Online] KARLSRUHE (DE) 16 August 2004 ZHU S. ET AL.: 'Precursors of three unique cysteine-rich peptides from the scorpion Buthus martensii Karsch', XP002994659 Retrieved from STN Database accession no. 137:151442 & COMPARATIVE BIOCHEMISTRY AND PHYSIOLOGY, PART B: BIOCHEMISTRY & MOLECULAR BIOLOGY vol. 131B, no. 4, 2002, pages 749 - 756
- HANADA K ET AL: "The Nonsynonymous/Synonymous Substitution Rate Ration versus the Radical/Conservative Replacement Rate Ration in the Evolution of Mammalian Genes" MOLECULAR BIOLOGY AND EVOLUTION, vol. 24, no. 10, 25 July 2007 (2007-07-25) , pages 2235-2241, DOI: 10.1093/molbev/msm152
- Karplus K: 9 February 2005 (2005-02-09), Retrieved from the Internet: URL:htpps://lists.sdsc.edu/pipermail/pdb-I /2005-February/002259.html> [retrieved on 2009-06-08]

## Description

### Field of the invention

The present invention relates to Cholesterol biosynthesis pathway modulators. In particular, to polypeptides having the function of HMGCoA reductase inhibitors, reducing the accumulation of cholesterol in the cholesterol biosynthesis pathway and/or reducing the level of serum cholesterol. The invention also relates to methods of treatment or prophylaxis of disorders characterised by the accumulation of cholesterol, its by-product and/or related lipid derived products, comprising administering to a subject any polypeptide of the invention.

### Background of the invention

Cholesterol is needed for cell membrane structure and as a precursor for bile acid and steroid hormones synthesis. Cholesterol is also required for cellular signalling pathway. Nevertheless, excess cellular free cholesterol is undesirable. Elevated plasma low density lipoprotein, (LDL)-cholesterol levels and low levels of high density lipoproteins (HDL)-cholesterol are known risk factors for the development and progression of atherosclerosis and coronary heart diseases (Brunzell and Hokanson, 1999, Am J Med 1999 Aug 23;107(2A):16S-18S; Tribble DL, Krauss RM., Adv Intern Med, 1993;38:1-29). Several epidemiological studies have also demonstrated that elevated plasma concentrations of triglycerids and low levels of HDL are a risk factor for the development of coronary heart diseases (Gotto AM Jr., Am J Manag Care 2002 Jan;Suppl:2, 4). HMG-CoA reductase (HMGR) plays an important and rate-limiting role in cholesterol biosynthesis. This enzyme is responsible for catalyzing the conversion of HMG-CoA to mevalonate in the cholesterol biosynthetic pathway and is the target of compounds that are very effective in lowering serum cholesterol.

Hyperlipidemic patients are treated with cholesterol lowering medications and particularly with inhibitors of 3-hydroxy-3-methylglutaryl coenzyme A (HMG-CoA) reductase. Statins are specific inhibitors of HMG-CoA reductase. These drugs have shown to lower the LDL-cholesterol level of patients and are the most effective and tolerated drugs currently used in the treatment of hypercholesterolemia (Brown et al, Am Heart J. 2002 Dec,144(6):1036-43).

Currently there are five statins, lovastatin, simvastatin, pravastatin, fluvastatin, and atorvastatin available. These drugs lower cholesterol by slowing down the production of cholesterol and by increasing the liver's ability to remove the LDL-cholesterol present in the blood. Studies have shown that about 20 to 60 percent lowering of LDL-cholesterol levels can be brought about in patients using these drugs.

Statins are also known to reduce triglyceride levels and cause an increase in HDL-cholesterol, as well as elevate the synthesis of LDL-receptors. However, different types of statins seem to show significantly different activities, either at the same or different doses (Serajuddin et al, J Pharm Sci 1991 Sep;80(9):830-4). They differ not only in their mode of action but also in their potency (Cohen LH, et al., Biopharm Drug Dispos 2000 Dec;21(9):353-64), metabolic rate, tissue selectivity/affinity, absorption (Nezasa K, et al., Drug Metab Dispos 2002 Nov;30(11):1158-63) and duration of action. Nevertheless, all the statins show lower incidence of adverse effects.

Accordingly, there is a need in the art for the availability of further and alternative drugs for reducing the level of serum cholesterol.

### Summary of the invention

The present invention addresses the problems above, and provides new cholesterol biosynthesis pathway modulators. In particular, the present invention provides polypeptides having the function of HMGCoA reductase inhibitors, reducing the accumulation of cholesterol in the cholesterol biosynthesis pathway and/or reducing the level of serum cholesterol. The invention also relates to methods of treatment or prophylaxis of disorders characterised by the accumulation of cholesterol, its by-products and/or related lipid derived products, comprising administering to a subject any polypeptide of the invention.

According to a first aspect, the invention provides an isolated nucleic acid molecule selected from the group consisting of:
(a) nucleic acid molecules consisting of the nucleotide sequence of SEQ ID NO:1;
(b) nucleic acid molecules encoding a peptide
   wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13;
(c) nucleic acid molecules consisting of a nucleotide sequence having at teast 80% identity with the sequence of SEQ ID NO:1 or a fragment thereof having at least 80% identity with the sequence of SEQ ID NO: 1 and which encode a peptide having the function of HMGCoA reductase inhibitor, reducing the accumulation of cholesterol in the cholesterol biosynthesis pathway and/or reducing the level of serum cholesterol.

The nucleic acid molecule (c) may be a nucleic acid molecule consisting of a nucleotide sequence having at least 80% or 90% identity with the sequence of SEQ ID NO:1 or a fragment thereof having at least 80% identity with the sequence of SEQ ID NO: 1 and which encodes a peptide having the function of HMGCoA reductase inhibitor, reducing the accumulation of cholesterol in the cholesterol biosynthesis pathway and/or reducing the level of serum cholesterol.

The nucleic acid molecule of the invention can be a single or double stranded polynucleotide, oligonucleotide, genomic DNA, cDNA, RNA, or mRNA.

The invention also provides a vector comprising at least one of the nucleic acid molecules (a), (b), (c). In particular, the vector may comprise at least two from the nucleic acid molecules (a), (b), and (c) are fused together.

The vector may further comprise a regulatory nucleic acid sequence, which is linked to the nucleic acid molecule encoding the polypeptide. The regulatory nucleic acid may be a prokaryotic or eukaryotic promoter.

The invention further provides a host cell comprising the vector. The host cell may be present in the form of a cell culture. The host cell may be a prokaryotic or eukaryotic cell.

In particular, the host cell is cultured to express a peptide wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13;

According to nother aspect the invention provides an isolated peptide, wherein the peptide is a fragment of SEQ ID NO:2 and comprises the amino acrid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13.

The peptide of the invention may be isolated and/or purified from biological material, expressed from recombinant DNA, and/or prepared by chemical synthesis.

In particular, the peptide may be isolated and/or purified from a human or non-human animal species.

According to another aspect, the peptide of the invention is isolated and/or purified from the venom. Any suitable venom from any animal comprising venom can be used. The peptide can be isolated and/or purified according to any standard technology known in the art. In particular, the peptide may be obtained from steps comprising:
- obtaining crude venom;
- carrying out gel filtration; and
- performing reverse-phase high performance liquid chromatography.

However, the method of isolating and/or obtaining the peptide of the invention may be any other method known in the art.

According to one example, the venom is from *Buthus martensii* Karsch, however, venom from other species may also be used. Accordingly, in particular, the peptide of the invention is isolated and/or purified from the step comprising:
- obtaining crude venom of a *Buthus martensii* Karsch;
- carrying out gel filtration;
- performing reverse-phase high performance liquid chromatography.

According to another aspect, the invention provides a pharmaceutical preparation comprising the peptide of the invention. The pharmaceutical preparation optionally comprises a pharmaceutically acceptable carrier, diluent, excipient or a combination thereof.

The pharmaceutical preparation may be in the form of oral, parenteral, injection, topical, and/or implant preparation.

The peptide or the pharmaceutical preparation may be administered locally, by injection, implantation, topical administration to a tissue locus, parenterally and/or orally. Other forms of administration known in the art and suitable for the purposes of the present use are also within the scope of the present invention.

According to another aspect, the invention provides a use of at least one peptide of the invention for the preparation of a medicament for the treatment or prophylaxis of disorders characterised by the accumulation of cholesterol, its by-product and/or related lipid derived products. In particular, the by-product comprises bile acid while related lipid derived products comprises HDL, LDL and/or VLDL. The disorders comprise hypertension, atherosclerosis, stroke, neurovascular and/or cardiovascular disorders.

According to a further aspect, the medicament further comprises a pharmaceutically acceptable carrier, diluent, excipient or a combination thereof. The medicament is administered locally, by injection, implantation, topical administration to a tissue locus, parenterally and/or orally.

Another aspect of the resent invention is a use of at least one peptide of the invention for the preparation of a medicament for the treatment for cholesterol independent and pleiotropic conditions. In particular, the cholesterol independent and pleiotropic condition comprise atherosclerotic stabilization, amelioration of endothelial dysfunction, improved coronary artery compliance, prevention of plaque rupture, and/or thrombus formation.

### Brief description of the figures

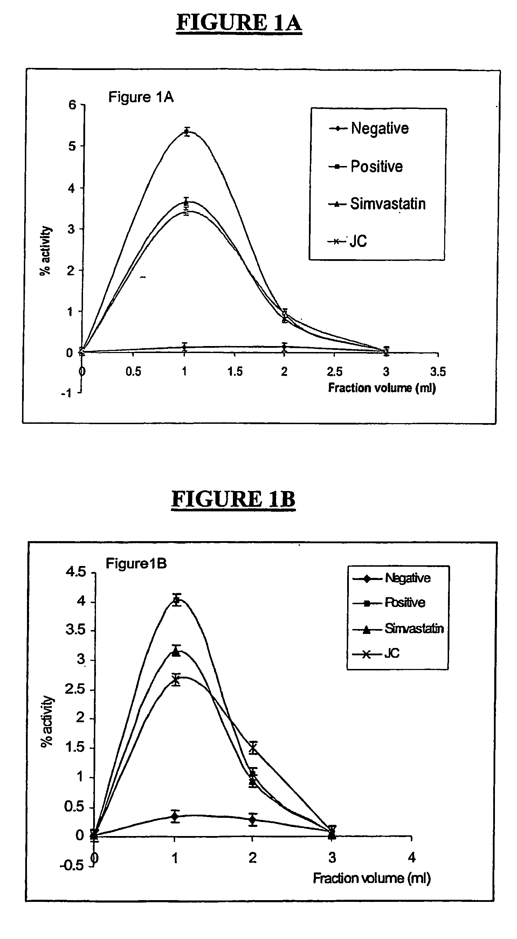

Figure 1(A and B): HMGCoA reductase activity of JC and Simvastatin using (a) rat hepatocyte and (b) HepG2 cell enzymes. Figure 1A: HMGCoA reductase assay, using the enzyme isolated from rat hepatocytes, using both simvasatin (10uM) and crude JC (10ug). The inhibitors were added to the enzyme assay. Figure 1B: HMGCoA reductase assay for HepG2 cells treated with simvastatin (10uM) and crude JC (10ug). The cells were then scraped and sonicated to break the cell for proteins. The cell proteins were used as enzyme source. In both cases, the positive controls were cells without any treatment while the negative controls were assays without the enzyme

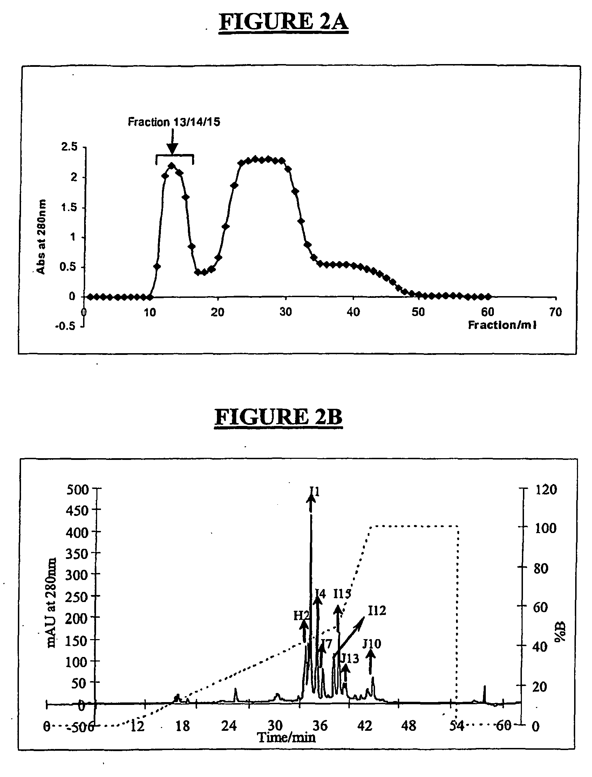

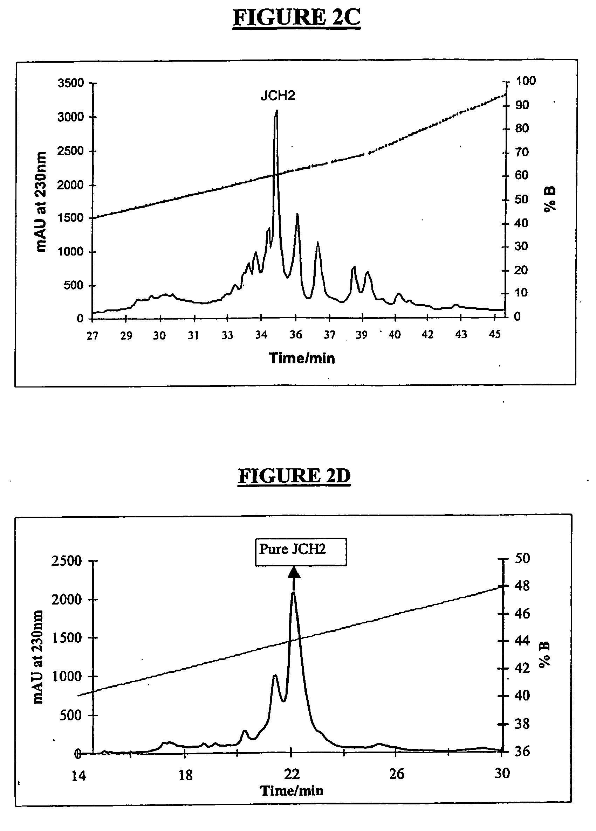

Figure 2(A, B, C. D): Gel filtration chromatography and RP-HPLC purifications of native JCH2 from *Buthus martensii* Karsch venom (a) Sephadex G-50 gel filtration chromatography of the crude venom; (b) RP-HPLC of the active fraction (13/14/15) from G-50 gel filtration on a Jupiter C18 column; (c) RP-HPLC of the active fraction (H2) from (b) on Jupiter C4 column; (d) final RP-HPLC purification of JCH2 on Jupiter C4 column from the active fraction (c).

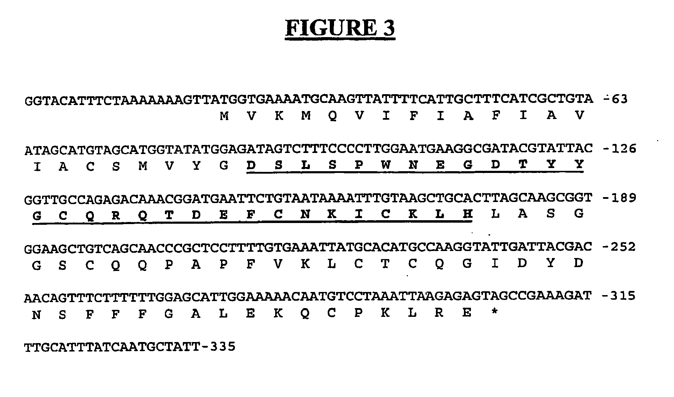

Figure 3: PCR based cDNA cloning was carried out. Primers were designed based on the determined N-terminal sequence of native JCH2. cDNA (SEQ ID NO:1) and deduced amino acid sequence of JCH2 (SEQ ID NO:2). The N-terminal sequence obtained from direct amino acid sequencing (SEQ ID NO:4) is undermined.

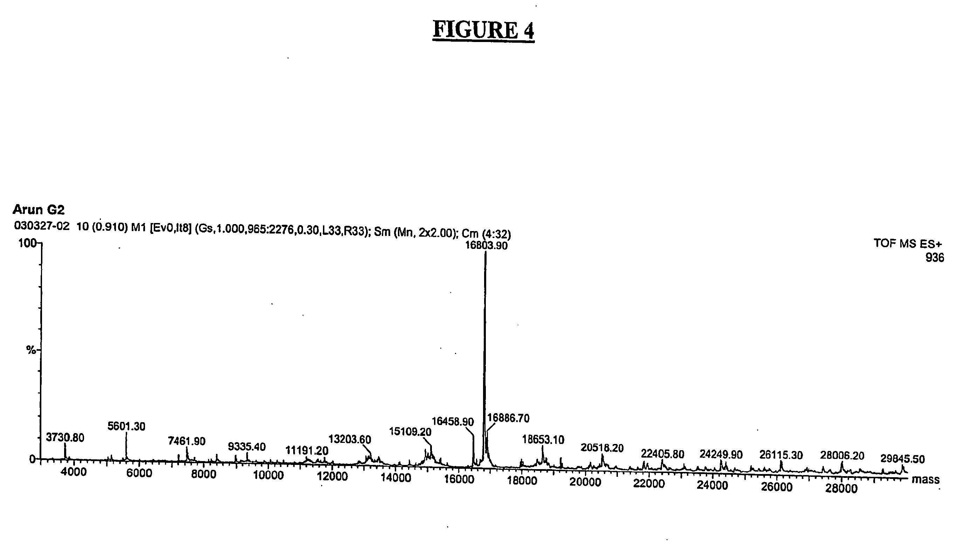

Figure 4: Mass analysis of JCH2 was carried out on a Q-TOF mass spectrophotometer (Micromass UK Ltd). The mass spectrum was processed using Micromass MassLynx software. The mass is determined to be 16803.9Da. The N-terminal sequencing (Edman Degradation) of JCH2 was carried out on Procise-HT protein sequencer attached to a 140C PTH analyser (Applied Biosystems, USA). The partial amino acid sequence were determined to be: DSLSPWNEGDTYYGCQRQTDEFCNKICKLH (SEQ ID NO:4).

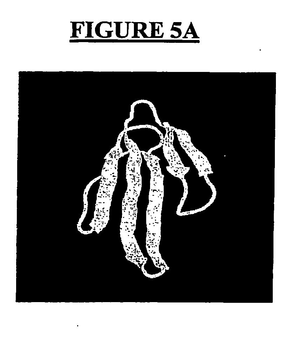

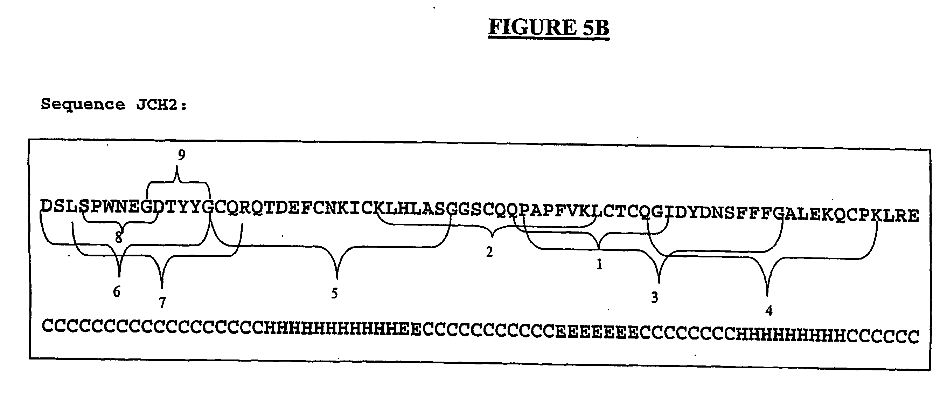

Figure 5(A. B): (a) The 3D structure of JCH2 was predicted based on homology modelling facility available on: (http://www.expasy.org/swissmod/SWISS-MODEL.html) using the first approach mode model and (b) the deduced amino acid sequence of JCH2 and the synthetic peptides derived from the sequence. The numbers refer to the individual peptide fragments (peptides 1-9) as shown in Table 2. Letter H, C and E refer to helix, coil and beta strand respectively.

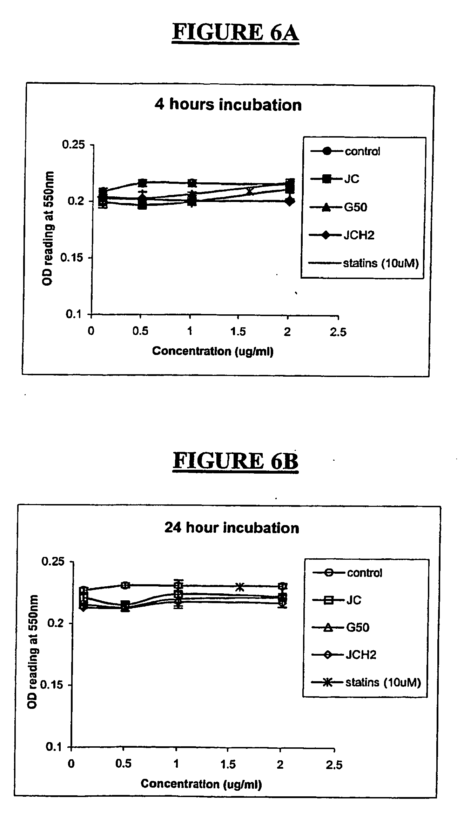

Figure 6(A, B): Cell viability (MTT) assay on HepG2 cell treated (a) 4 hours of incubation, (b) 24 hours of incubation, with various concentration of statins (10uM each), JC (crude venom), G50 (partially purified JC; Fig 2A) and JCH2 (pure HMGCoA reductase inhibitor from venom; Fig 2D). Viability (Abs at 550) was plotted against protein concentration of the inhibitors.

### Detailed description of the invention

The present invention provides new Cholesterol biosynthesis pathway modulators. In particular, the present invention provides polypeptides having the function of HMG-CoA reductase inhibitors; reducing the accumulation of cholesterol in the cholesterol biosynthesis pathway and/or reducing the level of serum cholesterol.

HMG-CoA (3-hydroxy-3-methylglutaryl-coenzyme A) reductase is the enzyme which catalyzes the rate limiting step of cholesterol biosynthesis. HMG-CoA reductase inhibitors, also known as statins, are molecules which inhibit the enzymatic activity of HMG-CoA reductase and have been used to treat patients suffering from hypercholesterolemia. The first such inhibitor (compactin or Mevastatin) was isolated in 1976 (Endo, A. et al., F.E. B.S. Lett., 72: 323-326, 1976) and since then many other natural and chemically modified versions of Mevastatin have been identified and developed for clinical use, including Lovastatin (Mevacor), and Simvastatin (Zocor).

Additonally, several studies have shown that HMG-CoA reductase inhibitors have anti-angiogenic activity. Feleszko, W. et. al., Int. J. Cancer, 81: 560-567 (1999) reported that treatment of a mouse model of tumour-cell induced angiogenesis with a combination of TNF-alpha and lovastatin produced a significant inhibition of tumor-induced blood-vessel formation whereas treatment with either TNF-alpha or lovastatin alone showed no angiostatic effects. Jones, M. K. et al., Am. J. Physiol., 276: G1345-GI1355 (1999) reported that mevastatin, an inhibitor of Ras activation, completely blocked the induction of VEGF (a potent angiogenic factor) expression in cultured primary endothelial cells. Kong, D. et al., Circulation, 100(18): I-39, Abstract #194 (1999) reported that simvastatin exerted potent anti-angiogenic effects independent of its cholesterol lowering effects.

Recent studies have shown that in addition to the treatment of hyperlipidemia, HMG-CoA reductase inhibitors are useful in the treatment of acne and/or skin aging (see, e.g. Breton, L. et al., US 5,902,805); can increase nitric oxide (NO)-mediated vasodilation and blood vessel relaxation (see e.g., Liao, J. K. et al., WO 99/18952); and can help prevent a second or additional myocardial infarction (see, e.g., Behounek, B. D. et al., US 5,674,893; Olukotun, A. Y. et al., US 5,622,985).

The regulation of serum cholesterol in mammals and particularly primates has attracted significant attention. It is often reported that regulation of cholesterol homeostasis in humans and other mammals involves regulation of cholesterol production, bile acid biosynthesis and catabolism of specific serum cholesterol carriers. Important serum cholesterol carriers are called LDL (low density lipoprotein) particles. The LDL receptor has been reported to facilitate internalization of the LDL particle into those cells in need of cholesterol. See e.g., Brown, M. S. and Goldstein, J. L. (1986) Science 232: 34-47; and Goldstein, J. L. and Brown, (1986) Nature, 348: 425; and references cited therein. The LDL receptor has been disclosed as impacting serum cholesterol levels in humans. For example, there has been recognition that cells with enough cholesterol do not make sufficient LDL receptors, thereby reducing or even blocking uptake of cholesterol by the cell. In this instance, serum cholesterol levels rise substantially, which can contribute to the development or severity of disease. Conversely, cells in need of cholesterol often have capacity to make more LDL receptors, thereby facilitating a decrease in serum cholesterol. Accordingly, there has been specific attention focused on regulating the LDL receptor as one therapeutic approach for stabilizing or reducing serum cholesterol levels in human patients.

Accordingly, the peptide according to the invention inhibits the cholesterol biosynthesis pathway by either binding directly and reducing the activity of HMG-CoA enzyme, or by binding to other enzymes at any possible step of the cholesterol biosynthesis pathway. A consequence of the reduction of the cholesterol biosynthesis is the reduction of serum cholesterol.

The peptide according to the invention, in particular the peptide JCH2, is capable of lowering the HMGCoA reductase activity at much lower concentrations than the known inhibitors, Fluvastatin, Mevastatin, Atorvastatin and Simvastatin, while exhibiting some beneficial effects which are different from that of the commonly used statins. Microarray analysis have also shown that besides reducing the reductase mRNA, the compound is also capable of lowering the mevalonate, the precursor for cholesterol biosynthesis The microarray data can be seen in Table 1. The RNA was isolated from HepG2 cell treated with the inhibitors and statins, that shows inhibitory activity on HMGCoA reductase assay. The global gene expression (DNA microarray) analysis was carried out on human gene chips (HG_U133A). The data displayed 67 genes that are either up- or down-regulated, upon treatment with venom component. Genes showing two-fold signal to log ratio in expression from two independent experiments were scored as significant.

**Table 1: Microarray Analysis (+: increase in expression, -: decrease in expression, NC: no change in gene expression compared to untreated control).**

| **Gene name** | **Simvastatin** | **JC** | **H2** |
|---|---|---|---|
| **1. Cholesterol metabolism** | | | |
| 203504_s_at ATP-binding cassette, sub-family A (ABC1), | NC | -1.7 | NC |
| member 1 | | | |
| **2. Calcium channel** | | | |
| 214933_at Calcium channel, voltage-dependent, | -1.6 | -1.9 | NC |
| P/Q type, alpha 1A subunit | | | |
| **3. Intracellular Signalling** | | | |
| 200605_s_at Protein kinase, | +2.1 | +1.8 | NC |
| cAMP-dependent regulatory, type 1, alpha | | | |
| **4. Small Molecule Transport** | | | |
| 201971_s_atATPase, H+transporting, | +1.8 | +2.3 | +2.5 |
| lysosomal 70kDa, V1 subunit A isoform 1 | | | |
| **5. Cell surface receptor linked signal transduction** | | | |
| 211000_s_at Interleukin 6 signal transducer | NC | +2.0 | +1.7 |
| (gp130, oncostatin M receptor) | | | |
| **6. Signal transduction** | | | |
| 217941 s at erbb2 interacting protein (ERBIN) | +2.0 | NC | NC |
| **7. Small Molecule Transport** | | | |
| 207408_at Organic cationic transporter-like 4 | NC | -1.4 | NC |
| 205421_at Solute carrier family | +3.5 | NC | +2.6 |
| (extraneuronal monoamine transporter), member 3 | | | |
| **8. Endothelial Recticulum** | | | |
| 207791 s_at RAB1A, member RAS oncogene family | +2.0 | +1.6 | NC |
| **9. Cell-cell matrix adhesion** | | | |
| 215177_s_at Integrin, alpha 6 | +3.6 | - | - |
| 214021 x at Integrin, beta 5 | +3.7 | NC | - |
| 10. 208995_s_at Peptidyl-prolyl isomerase G (cyclophilin G) | +2.2 | - | +2.9 |
| **11. Small Molecule Transport** | | | |
| 221487 s at Endosulfine alpha | +5.7 | - | - |
| **12. Mitochondrial** | | | |
| 200769 s at Methionine adenosyltransferase II, alpha | +2.1 | NC | - |
| **13. Smooth Muscle Contraction** | | | |
| 201617 x at Caldesmon 1 | +2.1 | NC | NC |
| **14. Carbohydrate metabolism** | | | |
| 202069 s at Isocitrate dehydrogenase 3 (NAD+) alpha | MI/+2.0 | NC | NC |
| **15. Chromosome condensation/segregation** | | | |
| 202131_s_at SudD suppressor of bimD6 homolog | +5.8 | NC | NC |
| (A. nidulans) | | | |
| **16. Transcription** | | | |
| 202817_s_at Synovial sarcoma translocation, chromosome | NC | NC | +2.5 |
| 18218129_s_at Nuclear transcription factor Y, beta | +2.3 | - | - |
| | | | |
| **17. Apoptosis** | | | |
| 203265 s at Mitogen-activated protein kinase kinase 4 | NC | NC | - |
| **18. Oncogenesis** | | | |
| 204394_at Prostate cancer overexpressed gene 1 | NC | -1.5 | NC |
| 208456_s at Related RAS viral (r-ras) oncogene homolog 2 | +2.0 | MI/+2.4 | NC |
| 211450 s at MutS homolog 6 (E. coli) | +2.1 | NC | NC |
| **19. Ossification** | | | |
| 208410_x_at Amelogenin (X chromosome, amelogenesis | -2.2 | NC | NC |
| imperfecta 1) | | | |
| | | | |
| **20. GPCRs, Class B Secretin-like** | | | |
| 208593 x at Corticotropin releasing hormone receptor 1 | NC/-2.0 | A/-2.3 | - |
| **21. Heat shock response** | | | |
| 208744 x at Heat shock 105kD | +5.6 | - | - |
| **22. mRNA splicing** | | | |
| 212885_at M-phase phosphoprotein 10 | MI/+2.6 | NC | NC |
| (U3 small nucleolar ribonucleoprotein) | | | |
| 222026 at RNA binding motif protein 3 | NC | NC | +1.7 |
| **23. Ubiquitin-dependent protein degradation** | | | |
| 209943 at F-box and leucine-rich repeat protein 4. | MI/+1.5 | NC | - |
| **24. Cell surface receptor linked signal transduction** | | | |
| 211339 s at IL2-inducible T-cell kinase | NC | NC | NC |
| **25. Cell shape and cell size control** | | | |
| 214925_s_at Spectrin, alpha, non-erythrocytic 1 (alpha- | NC | NC | NC |
| fodrin) | | | |
| **26. Others** | | | |
| 201151_s_at Muscleblind-like (Drosophila) | +2.3 | NC | NC |
| 202375_at SEC24 related gene family, member D | +1.5 | NC | NC |
| (S. cerevisiae) | | | |
| 202840_at TAF15 RNA polymerase II, | MD/-1.5 | -2.3 | NC |
| TATA box binding protein (TBP)-associated factor, 68kDa | | | |
| 205305_at Fibrinogen-like 1 | NC | -2.6 | NC |
| 206826_at Peripheral myelin protein 2 | NC | - | +2.7 |
| 210941_at BH-protocadherin (brain-heart) | - | NC | +3.4 |
| 211521_s_at Pleckstrin homology, Sec7 and coiled/coil | - | - | - |
| domains 4 | | | |
| 213297_at Nucleolar protein family A, member 2 | NC | NC | NC |
| (H/ACA small nucleolar RNPs) | | | |
| 213534_s_at PAS domain containing serine/threonine kinase | NC | NC | +2.1 |
| 215743_at Ribonuclease P (38kD) | NC | NC | +2.1 |
| 215954_s_at NY-REN-24 antigen | +2.8 | NC | - |
| 217383_at Phosphoglycerate kinase {alternatively spliced} | NC | -1.2 | NC |
| [Human, phosphoglycerate kinase deficient | | | |
| patient | | | |
| with episodes of muscl, mRNA Partial Mutant, | | | |
| 307 | +2.2 | - | - |
| nt] | | | |
| 221618_s_at TAF9-like RNA polymerase II, | +1.6 | NC | NC |
| TATA box binding protein (TBP)-associated factor, 31 kDa | | | |
| 222119 s at Vitiligo-associated protein VIT-1 | | | |

In particular, JCH2 has proved to be a potent inhibitor of HMGCoA reductase in comparison to statins, where the percentage of inhibition of 0.6µM JCH2 is similar to that of 10µM statins (Figure 1A and 1B, and Table 2). Further analysis (gene expression and protein profiling) has shown that the JCH2 brings about the inhibitory effect possibly *via* a different pathway than for the existing statins. Both the microarray and the protein profiling data are shown in Table 1 and Table 3. As for the protein profiling data, the profiles as shown in Table 3 were obtained by surface-enhanced laser absorption ionization-time of flight mass spectro-photometry (SELDI-TOF) on treated and control samples using the H4 (hydrophobic) and the NP20 (hydrophilic) chips. Comparison of the Simvastatin treated HepG2 cell protein profile with that of JC or JCH2 treated cells changes shows the specific proteins that are either enhanced or repressed upon treatment with the venom component.

According to a first aspect, the invention provides an isolated nucleic acid molecule selected from the group consisting of:
(a) nucleic acid molecules consisting of nucleotide sequence of SEQ ID NO:1;
(b) nucleic acid molecules encoding a peptide,
   wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13;
(c) nudeic acid molecules comprising a nucleotide sequence having at least 80% identity with the sequence of SEQ ID NO:1 or a fragment thereof having at least 80% identity with the sequence of SEQ ID NO:1 and which encode a peptide having the function of HMGCoA reductase inhibitor, phosphomevalonate inhibitor, reducing the accumulation of cholesterol in the cholesterol biosynthesis pathway and/or reducing the level of serum cholesterol.

The present invention provides a peptide (protein) of SEQ ID NO:2 that inhibits the HMGCoA reductase activity. As used herein, the term fragment of the peptide is defined by its ability to inhibit HMGCoA reductase, reducing the accumulation of cholesterol in the cholesterol biosynthesis pathway and/or reducing the level of serum cholesterol. With reference to the HMGCoA reductase activity, it can be measured by the assay described in the examples or by other assays available in the art. Accordingly, the present invention also provides a nucleic acid molecule (a) consisting of the sequence of SEQ ID NO:1, and nucleic acid molecules (b), and/or (c) as indicated above.

A nucleic acid according to the invention may be a single or double stranded oligonucleotide or polypeptide, or can also be single or double stranded genomic DNA, cDNA, RNA, mRNA. In the case in which a nucleic acid molecule is transcribed and translated to produce a functional peptide, defined in this case as a peptide that retains HMGCoA reductase, reducing the accumulation of cholesterol in the cholesterol biosynthesis pathway and/or reducing the level of serum cholesterol, one of skill in the art recognizes that because of codon degeneracy a number of different nucleic acids encode the same peptide.

A fragment of the peptide of SEQ ID NO:2 are the amino acid sequences SEQ ID NOS:3-13, reported in Table 2. Accordingly, the nucleic acid molecule (b) is a nucleic acid molecule encoding at least a peptide consisting of the amino acid sequence of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13. These sequences and their ability in reducing HMG-CoA reductase activity are reported in Table 2.

Further, homologues of the peptide in SEQ ID NO:2 of the invention are peptides that are "substantially identical" to the peptide in SEQ ID NO:2, retain the inhibitory activity and are found in or isolated from different species of animals than the peptide in the present invention, which is human in origin.

Homologues of the peptide in SEQ ID NO:2 are defined by their ability to inhibit the HMGCoA reductase, reduce the accumulation of cholesterol in the cholesterol biosynthesis pathway and/or reduce the level of serum cholesterol in the progression of cholesterol biosynthetic pathway or their by-products. The critical amino acids in a protein or peptide are those amino acids that are important for the function of the protein or peptide. These amino acids are often conserved across different species and organisms. It is well known to one skilled in the art that conserved amino acids are usually identical across species and organisms and if not, are very similar in their chemical properties. Homologues of the peptide in SEQ ID NO:2 are those peptides that are found in other species and organisms that inhibit HMGCoA reductase, reduce the accumulation of cholesterol in the cholesterol biosynthesis pathway and/or reduce the level of serum cholesterol. Homologues of the peptide in SEQ ID NO:2 will contain the conserved amino acids that are critical to the function of the peptide. These amino acids are either identical to the corresponding amino acids in SEQ ID NO:2 or have very similar chemical properties.

Accordingly, the nucleic acid molecule (c) may be a nucleic acid molecule comprising a nucleotide sequence having at least 80% or 90% identity with the sequence of SEQ ID NO:1 or a fragment thereof and which encodes a peptide having the function of HMGCoA reductase inhibitor, phosphomevalonate inhibitor, reducing the accumulation of cholesterol in the cholesterol biosynthesis pathway and/or reducing the level of serum cholesterol. In particular, the nucleic acid molecule (c) has at least 80% identity with the sequence of SEQ ID NO:1 or a fragment thereof, preferably at least 90%.

Further homologs showing inhibition on HMGCoA reductase activity, reducing the accumulation of cholesterol in the cholesterol biosynthesis pathway and/or reducing the level of serum cholesterol, can be obtained from venom of any known species. For example, homologs obtained from the same venom of *Buthus martensii* Karsch, and showing inhibition on HMGCoA reductase activity, reducing the accumulation of cholesterol in the cholesterol biosynthesis pathway and/or reducing the level of serum cholesterol, are reported in Table 2. The molecular weights (masses) of the obtained homologs are the following: 16790Da (JC1-P1); 16791 Da (JC2-P1) and 17211 Da (JC3-P5) (see Table 2).

The invention also provides a vector comprising at least one of the nucleic acid molecules (a), (b), (c). In particular, the vector may comprise at least two from the nucleic acid molecules (a), (b), and (c) fused together. The vector may further comprise a regulatory nucleic acid sequence linked to the nucleic acid molecule encoding the polypeptide. The regulatory nucleic acid may be a prokaryotic or eukaryotic promoter.

The invention further provides a host cell comprising the vector. The host cell may be present in the form of a cell culture. The host cell may be a prokaryotic or eukaryotic cell.

In particular, the host cell is cultured to express a peptide of the invention.

Methods for the preparation of a vector comprising a nucleic acid molecule according to the invention, preferably linked to a promoter, and cultivation of the host cell comprising the vector can be carried out according to standard techniques. For example, as indicated or described in Sambrook and Russel, Molecular Cloning, Cold Spring Harbour, 2002.

According to another aspect, the invention provides an isolated peptide, having the function of HMGCoA reductase inhibitor, phosphomevalonate inhibitor, reducing the accumulation of cholesterol in the cholesterol biosynthesis pathway and/or reducing the level of serum cholesterol.
wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13;

The peptide of the invention may be isolated and/or purified from biological material, expressed from recombinant DNA, and/or prepared by chemical synthesis.

In particular, the peptide may be isolated and/or purified from a human or non-human animal species.

According to another aspect, the peptide of the invention is isolated and/or purified from the venom. Any suitable venom from any animal comprising venom can be used. The peptide can be isolated and/or purified according to any standard technology known in the art. In particular, the peptide may be obtained from steps comprising:
- obtaining crude venom;
- carrying out gel filtration; and
- performing reverse-phase high performance liquid chromatography.

However, the method of isolating and/or obtaining the peptide of the invention may be any other method known in the art.

According to another aspect, the peptide of the invention is isolated and/or purified to homogeneity from the venom of *Buthus martensii* Karsch, which is a Chinese scorpion, however, venom from other species may also be used. Accordingly, in particular, the peptide of the invention is isolated and/or purified from the step comprising:
- obtaining crude venom of a *Buthus martensii* Karsch;
- carrying out gel filtration;
- performing reverse-phase high performance liquid chromatography (RPHPLC).

The peptide isolated form the crude venom of *Buthus martensii* Karsch is indicated as JCH2 protein or peptide.

In particular, the gel filtration step may be a sequential gel filtration. However, the method for isolation and/or purification of a peptide according to the invention, in particular the peptide JCH2 is not limited to that exemplified herein. Any suitable method known in the art may also be used.

The protein JCH2 showed higher inhibitory activity than the Simvastatin, Mevastatin, Atorvastatin and Fluvastatin when tested for HMGCoA reductase activity on HepG2 cell purified HMGCoA reductase as well as enzymes purified from the rat liver microsomes (Fig.1A and 1B). The protein sequence of JCH2 is hereafter referred to as SEQ ID NO:2. The protein has been purified to homogeneity and characterized. The cDNAs encoding JCH2 (SEQ ID NO:1) have been cloned from the venom gland (telson) mRNA. Series of synthetic peptides (SEQ ID NOS:3-13) have also been made and their inhibitory properties were tested.

According to another aspect, the invention provides a pharmaceutical preparation comprising the peptide of the invention, in particular the peptide JCH2 or a homologue, variant, derivative or fragment thereof. The pharmaceutically preparation optionally comprises a pharmaceutically acceptable carrier, diluent, excipient or a combination thereof. The invention encompasses the preparation and use of pharmaceutical compositions comprising the peptide of the invention, in particular the peptide JCH2 or a homologue, variant, derivative or fragment as an active ingredient. Such a pharmaceutical composition may consist of the active ingredient alone, in a form suitable for administration to a subject, or alternatively the pharmaceutical composition may comprise the active ingredient and one or more pharmaceutically acceptable carrier, excipient and/or diluent.

The pharmaceutically preparation may be in the form of oral, parenteral, injection, topical, and/or implant preparation.

Pharmaceutical compositions of peptides may be used to inhibit the HMGCoA reductase, for example, in th treatment or prophylaxis of disorders characterized by the accumulation of cholesterol or its by-product (bile acid) or related lipid derived products (such as HDL, LDL, VLDL etc) such as, for example, hypertension, atherosclerosis, stroke, as well as neurovascular and cardiovascular disorders or in cholesterol independent or pleiotropic effects which could include atherosclerotic stabilization, amelioration of endothelial dysfunction, improved coronary artery compliance and prevention of plaque rupture and thrombus formation.

Compositions of the invention are provided to an animal by any suitable means, directly (e.g., locally, as by injection, implantation or topical administration to a tissue locus) or systemically (e.g., parenterally or orally). Where the compositions of the invention are to be provided parenterally, such as by intravenous, subcutaneous, opthalmic, intraperitoneal, intramuscular, buccal, rectal, vaginal, intraorbital, intracerebral, intracranial, intraspinal, intraventricular, intrathecal, intracisternal, intracapsular, intranasal or by aerosol administration, the composition preferably comprises part of an aqueous or physiologically compatible fluid suspension or solution.

According to another aspect, the disclosure includes methods for modulating and particularly reducing serum cholesterol level in a mammal. In this embodiment, the methods generally include administering to the mammal a therapeutically effective amount of at least one and typically one of peptide comprising the amino acid sequence of SEQ ID NO: 2, SEQ ID NO:3, SEQ ID NO:4, SEQ ID NO:5, SEQ ID NO:6, SEQ ID NO:7, SEQ ID NO:8, SEQ ID NO:6, SEQ ID NO:10, SEQ ID NO:11, SEQ ID NO:12 and SEQ ID NO:13. Further, the method of the disclosure may also comprise administering a pharmaceutical composition according to the invention.

Cholesterol is generally understood to be essential for normal growth and viability of most higher organisms. Too much serum cholesterol has been correlated with life threatening lipid-related diseases including hyperlipoproteinemia, stoke, coronary heart disease, and especially atherosclerosis and related conditions. See generally Stryer, L. (1988) in Biochemistry, 3.sup.rd Ed. W. H. Freeman and Co. New York, pp. 547-574; and Brown, M. S. and Goldstein, J. L. (1993) in The Pharmacological Basis of Therapeutics (8.sup.th Ed.) Gilman, A. G. et al. eds. McGraw-Hill/New York, pp. 874-896.

According to another aspect, the disclosure provides a method of treatment or prophylaxis of disorders characterised by the accumulation of cholesterol, its by-product and/or related lipid derived products, comprising administering to a subject in need at least one peptide of the invention having the function of HMGCoA reductase inhibitor, reducing the accumulation of cholesterol in the cholesterol biosynthesis pathway and/or reducing the level of serum cholesterol. The method may also comprise administering at least one peptide or a fused peptide comprising the amino acid sequence of at least one of SEQ ID NO:2, SEQ ID NO:3, SEQ ID NO:4, SEQ ID NO:5, SEX ID NO:6, SEQ ID NO:7, SEQ ID NO:8, SEQ ID NO:9, SEQ ID NO:10, SEQ ID NO:11, SEQ ID NO:12 and SEQ ID NO:13. Further, the method of the disclosure may also comprise administering a pharmaceutical composition according to the invention.

The by-product comprises bile acid. The related lipid derived products comprises HDL, LDL and/or VLDL.

The disorders comprise hypertension, atherosclerosis, stroke, neurovascular and/or cardiovascular disorders.

The peptide or the pharmaceutical preparation may be administered locally, by injection, implantation, topical administration to a tissue locus, parenterally and/or orally. Other forms of administration known in the art and suitable for the purposes of the present method are also within the scope of the present invention.

Apart from their lipid lowering actions, a direct cholesterol dependent activity, statins are proposed to have other important cholesterol-independent or pleiotropic effects including atherosclerotic stabilization, amelioration of endothelial dysfunction, improved coronary artery compliance and prevention of plaque rupture and thrombus formation (Takemoto and Liao 2001, Arterioscler Thromb Vasc Biol 2001 Nov;21(11):1712-9; Liao, 2002, J Clin Invest 2002 Aug;110(3):285-8; Vaughan, 2003, Am J Cardiol 2003 Feb 20;91(4A):23B-29B).

Accordingly, the disclosure also provides a method of treatment for cholesterol independent and pleiotropic conditions comprising administering to a subject at least one peptide of the invention. The method may also comprise administering at least one peptide comprising the amino acid sequence of at least one of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID N0:11, SEQ ID NO:12 and SEQ ID NO:13.

The cholesterol independent and pleiotropic condition comprise, but are not limited to, atherosclerotic stabilization, amelioration of endothelial dysfunction, improved coronary artery compliance, prevention of plaque rupture, and/or thrombus formation.

According to another aspect of the present invention is a use of at least one peptide of the invention having the function of HMGCoA reductase inhibitor, reducing the accumulation of cholesterol in the cholesterol biosynthesis pathway and/or reducing the level of serum cholesterol, for the preparation of a medicament for the treatment or prophylaxis of disorders characterised by the accumulation of cholesterol, its by-product and/or related lipid derived products.

In particular, the by-product comprises bile acid and related lipid derived products comprises HDL, LDL and/or VLDL. Further, the disorders comprise hypertension, atherosclerosis, stroke, neurovascular and/or cardiovascular disorders.

The medicament may comprise at least one peptide comprising the amino acid sequence of at least one of SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 and SEQ 10 NO:13. The medicament further comprises a pharmaceutically acceptable carrier, diluent, excipient or a combination thereof. The medicament may be administered locally, by injection, implantation, topical administration to a tissue locus, parenterally and/or orally.

A further aspect of the present invention is the use of at least one peptide of the present invention for the preparation of a medicament for the treatment for cholesterol independent an pleiotropic conditions. Cholesterol independent and pleiotropic condition comprise atherosclerotic stabilization, amelioration of endothelial dysfunction, improved coronary artery compliance, prevention of plaque rupture, and/or thrombus formation.

The medicament may also comprise at least one peptide or a fused peptide comprising the amino acid sequence of SEQ ID NO:3.

Having now generally described the invention, the same will be more readily understood through reference to the following examples, which are provided by way of illustration, and are not intended to be limiting of the present invention.

### Examples

### Example 1

### Human Hepatoma cell line-HepG2 cell culture and treatment

The cell stocks of Human Hepatoma cell line-HepG2 were cultured in T-25 flask in Eagle's minimum essential medium (MEM), supplemented with 10% fetal bovine serum, and incubated in a humidified incubator (5% CO₂) at 37°C. At 70-80% confluent, treatment was started with 12 hours incubation in complete media supplemented with delipidated FBS (Gibson et al, J Lipid Res. 1990 Mar;31(3):515-21) This was followed by 4 hours of incubation in complete media with delipidated FBS as well as the inhibitors, statins and JCH2.

After 4 hours incubation with the inhibitors, the media was discarded and cells were washed with 0.90%saline. The cells were scraped and collected into a 50ml tube. The cells were centrifuged at 2400rpm for 5 min at 4 C, and the resulting cell pellet was washed twice with 10ml saline. The final cell pellet was resuspended in 30µl lysis buffer and briefly sonicated. The suspension was centrifuged at 14,000rpm for 15min and the supernatant containing the enzyme HMGCoA reductase, was used for the assay. Protein concentration was measured by the Bradford method (Bradford M 1976; Anal. Biochem. 72:248-254).

### HMGCoA reductase activity assay

In biosynthesis of cholesterol, HMG-CoA reductase is the key enzyme to convert HMG-CoA to its product mevalonate. In the presence of HCl, the mevalonate is converted into mevalonolactone.

[¹⁴C] HMG-CoA + HMG-CoA reductase → [¹⁴C] mevalonate

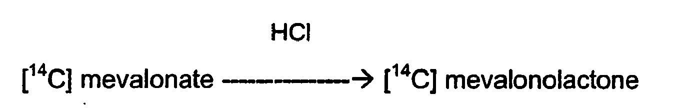

QAE-Sephadex A25 is an anionic-exchange column chromatography (K. K. Ong et al, 1991). It will retain HMG-CoA, which is negatively charge in the column but not mevalonolactone, which will be eluted out by ammonium formate.

The 50µl assay buffer (100mM potassium dihydrogen phosphate, 4mM dithiothreitol, 20mM glucose-6-phosphate, 20 unit glucose-6-phosphate dehydrogenase, 2.5mM NADP⁺, 30µM [14C] HMG-CoA (0.01µCi) and microsomal protein) was incubated at 37°C for 1 hour. Reaction was terminated by addition of 25 µl of 2M HCl. After 30min lactonization of mevalonate to mevalonolactone, 500 µl of 20mM ammonium formate was added to each sample, spun for 5 min at 14,000rpm.

Supernatant was applied to the column that is pre-packed with QAE-Sephadex (4cm), equilibrated with ammonium formate. The first 5 ml fractions was eluted with 20mM ammonium formate, followed by 5 ml of 0.05M HCl and 10ml of 0.1 M HCl. Radioactivity of the first 5ml (1ml fraction) was determined using Beckman LS6500 scintillation counter.

### MTT cell viability assay

MTT test is a colorimetric assay system (Sigma, USA) which measures the reduction of 3-(4,5- dimethylthiazole-2-yl)-2,5-diphenyl tetrazolium bromide(MTT) into an insoluble formazan product by activity of the mitochondrial enzymes in viable cells. MTT was dissolved in culture medium to a concentration of 0.5mg/ml and filtered. A 96well plate was seeded with 5000 cells per well and allowed to grow for 24 hours to 80% confluence. Duplicates were treated with statins and JC fractions. Incubation was carried out for 24 hours. The medium was removed, cells washed twice with 1x saline and then MTT (60µl) was added. Incubation was carried out for 1 hour and the MTT/medium was removed. The formazan crystals formed were solubilized in 250ul of dimethylsulfoxide (DMSO). Absorbance was measured in a multiwell scanning spectrophotometer at 550nm wavelength (Figure 6). Cell viability (MTT) assay was performed on HepG2 cell treated (a) 4 hours of incubation, (b) 24 hours of incubation, with various concentration of statins (10uM each), JC (crude venom), G50 (partially purified JC; Fig 2A) and JCH2 (pure HMGCoA reductase inhibitor from venom; Fig 2D. The viability (Abs at 550) was then plotted against protein concentration of the inhibitors.

### HMGCoA reductase activity of venom

Venom from Chinese scorpion (*Buthus martensi Karsch*) was tested for HMGCoA reductase activity using Simvastatin, Atoravstatin, Mevastatin and Fluvastatin as a control for inhibition. Protein concentration of 1-100µg/ml was used. The inhibitory activity was observed for statins as well as for the scorpion venom (Figure 1A). Figure 1A shows the results of the HMGCoA reductase assay, using the enzyme isolated from rat hepatocytes, using both simvastatin (10µM) and crude JC (10µg). The inhibitors were added to the enzyme assay. Figure 1B shows the results from the HMGCoA reductase assay for HepG2 cells treated with simvastatin (10µM) and crude JC (10µg). The cells were then scraped and sonicated to break the cell for proteins. The cell proteins were used as enzyme source. In both cases (Figure 1A and 1B), the positive controls were cells without any treatment while the negative controls were assays without the enzyme.

### Fractionation of Buthus martensii Karsch crude venom by gel filtration

The Chinese scorpion venom was chromatographed on Sephadex G-50 gel filtration using 10mM Tris-Cl pH 7.6. Figure 2a shows the elution profile of the crude venom. The HMGCoA reductase inhibition was seen for fractions from peak 1, P1, at 10 µg protein. Based on this result, P1 fractions (13, 14, 15), were subjected to reverse phase HPLC (RP-HPLC) on a C18 column (Jupiter C18, 5um; 4.6x250mm). The column was equilibrated with 0.1%TFA in H₂O and the protein was eluted using a step gradient of 0-20%B, followed by 20-70%B and finally 70%-100% B (0.05%TFA in 80% acetonitrile; Figure 2b). All the fractions from RP-HPLC were assayed for the HMGCoA reductase activity at 10µg protein. The H2 fraction showing high inhibitory activity was further purified on RP-HPLC (Figure 2c). The H2 fraction were further purified on Jupiter C4 column and tested for inhibitory property (Figure 2d).

### Cloning of the JCH2 from Buthus martensii Karsch

Total RNA extracted (Chomczynski, P. & Sacchi, N., 1987, Anal. Biochem. 162, 156-159) from the telsons, was reversed transcribed (Armugam, A., et al., 1997, Toxicon 35, 27-37.26). The double stranded cDNA was synthesised and ligated to universal adaptor (AP) on both ends. The adapted cDNAs were used as a source for amplifying specific clones using the gene specific primers forward 1:
5'GA(T/C)AG(T/C)CTNTCNCC(T/C)TGGAA(T/C)GA3' (SEQ ID NO:14) (with the form "(T/C)" it is indicated that in that position the nucleotide can be T or C);
and universal adaptor primer as reverse primer, AP1:
   5' GTAATACGACTCACTATAGGGC 3' (SEQ 10 NO:15).

A similar reaction was carried out using a gene specific reverse primer
5'ATTCCAAGG GGAAAGACTATC 3' (SEQ ID NO:16)
and the adaptor primer, AP1, as the forward primer. Both the PCR products were sequenced and the cDNA encoding JCH2 was amplified by PCR using the following gene specific primers: forward primer
5' GGTACATTTCTAAAAAAAGTTATG 3' (SEQ ID NO:17) and reverse primer:
5' AATAGCATTGATTAAATGCAAATC 3' (SEQ IDNO:18).

An approximately 400bp fragment of PCR product was obtained and subcloned. Putative positive clones were subjected to DNA sequencing (Sanger, F., et al., 1977, Proc. Natl. Acad. Sci. USA 74, 5463-5467; Armugam *et al*, 1997) on an automated DNA sequencer (Model 3100, Applied Biosystems, USA). All clones showed the presence of cDNA encoding JCH2 (Figure 3). The cDNA encoded for a 22 amino acid signal peptide and 72 amino acid residues of the mature protein (Figure 3).

The proteins have been found to be negatively charged with pl value (5.03) corresponding to acidic proteins. Interestingly, the mass of 16kDa (obtained from protein analysis) does not correlate to the mass deduced from cDNA, The mass deduced from the cDNA sequence is 8132Da while the mass of native protein was determined to be 16803Da. The JCH2 cDNA, showed that the JCH2 contains 7 Cys residues. Thus, only 3 disulphide bridges have been predicted for this protein. However the extra Cys residue might be involved in forming a covalent bonded dimer, as observed in the mass analysis (16803Da).

### Characterization of JCH2 protein

The purified native JCH2 was subjected to Mass spectrophotometer analysis. Mass spectrometry analyses were carried out on a Q-TOFF mass spectrophotometer (Micromass UK Ltd). The processing of the mass spectra was performed using the Micromass MassLynx software according to the manufacturer's protocol. The mass was determined to be 16803Da (Figure 4). The N-terminal amino acid sequence of this protein was determined to be-DSLSPWNEGDTYYGCQRQTDEFCNKICKLH- (SEQ ID NO:4) for the first 30 amino acid residues.

Homology modelling was performed to predict the tertiary structure of JCH2 (Figure 5A and 5B). Synthetic peptides were made based on the amino acid sequence of JCH2 and further tested for the HMGCoA reductase inhibitory activity (Figure 5A and 5B). All the HMGCoA reductase activity obtained for the protein fractions, statins and the synthetic peptides are presented in Table 2.

**Table 2: HMGCoA reductase activity of protein, statins and synthetic peptides. HepG2 cells were treated with native proteins at 10ug each [crude venom, JCG50 (Fig 2A), purified JCH2 (Fig 2D)], statins at 10uM each (Atorvastatin, Fluvastatin, imvastatin and Mevastatin) and the synthetic peptides at 10uM (derived from SEQ ID NO:3). The peptides and statins were dissolved in either DMSO or sterile water. Percentage of inhibition was calculated based on positive control (untreated cells): The sequences of homologs to JCH2 have the followings molecular weights (masses): 16790Da (JC1-P1); 16791Da (JC2-P1) and 17211Da (JC3-P5).**

| | **Description / Sequence of protein & peptides** | **% of inhibition on HMG-CoA** reductase, activity | |
|---|---|---|---|
| | | **Aqueous medium** | **DMSO medium** |
| **Crude venom (10µg)** | | 21% | - |
| **JCG50-P1(10**µ**g)** | Peak 1 from Sephadex G50 chromatography | 25% | |
| **JCH2 (0.6µM)** | Native protein (RP-HPLC) | 30% | - |
| **SEQ ID NO:3** | DSLSPWNEGDTYYGCQRQTDEFCNKICKLHLASG GSCQQPAPFVKLCTCQGIDYDNSFFFGALEKQCPK LRE | | |
| **JC1-P1** | Similar mass with JCH2 (16790Da for JC1-P1; 16791Da for JC2-P1 and 17211Da for JC3-P5) | 23% | |
| **JC2-P1** | | 25% | |
| **JC3-P5** | | 20% | |
| Peptide 1 | QPAPFVKLCTCQGI **(SEQ ID NO:5)** | 20% | 20% |
| **Peptide 2** | KLHLASGGSCQQPAPFVKL **(SEQ ID NO:6)** | 30% | 7% |
| **Peptide 3** | PAPFVKLCTCQGIDYDNSFFFG **(SEQ ID NO:7)** | 10% | 15% |
| **Peptide 4** | QGIDYDNSFFFGALEKQCPK **(SEQ ID NO:8)** | 7% | - |
| **Peptide 5** | GCQRQTDEFCNKICKLHLASG **(SEQ ID NO:9)** | 2% | - |
| **Peptide 6** | DSLSPWNEGDTYYG **(SEQ ID NO:10)** | 4% | - |
| **Peptide 7** | LSPWNEGDTYYGCQR **(SEQ ID NO:11**) | 2% | - |
| **Peptide 8** | SPWNEGD **(SEQ ID NO:12)** | 1% | - |
| **Peptide 9** | GDTYYG **(SEQ ID NO:13)** | 3% | - |
| **Atorvastatin (hydrophobic)** | An HMG-CoA reductase inhibitor, reduces total cholesterol, LDL and apolipoprotein B. | Insoluble in aqueous medium | 70% |
| **Fluvastatin (hydrophobic)** | A synthetic, competitive inhibitor of HMG-CoA reductase that acts as anti-hypercholesterolemic agent,reduces the basal MMP-1 levels in culture media of endothelial cells | Insoluble in aqueous medium | 74% |
| **Simvastatin (hydrophilic) (10µM)** | A lipophilic HMG-CoA reductase inhibitor that blocks Ras function and inhibits cell proliferation of human smooth muscle cells | **35%** | 65% |
| **Mevastatin (hydrophilic)** | Compactin: an antibiotic that acts as a potent inhibitor of HMG-CoA reductase, suppresses Ras farnesylation. Causes cell cycle arrest in late G1 phase | 35% | 60% |

| | | | |
|---|---|---|---|
| Note:Concentrations of all peptides and statins are 10µM except for JCH2 which is at 0.6µM (10µg) | | | |

**Table 3: Protein profiling data of hydrophobic chip, H4 (Table 3A) and hydrophilic chip, NP20 (Table 3B).**

| **Table 3A** | | | | |
|---|---|---|---|---|
| **Protein** | **Molecular weight (kDa)** | **Treatment** | | |
| | | **Sim** | **JC** | **JCH2** |
| ApoC-I | 6.6 | ↑↑ | ↓↓ | ↓ |
| ApoC-II / III | 8.8 | ↑ | ↓↓ | ↑↑ |
| ApoC-IV, SAA-1 protein | 11.0 | ↓ | ↑ | ↓ |
| sPLA₂, FABP (aP2) | 14.0 | ↓ | ↑ | ↓ |
| StAR related lipid transfer protein | 23.0 | ↑ | ↓ | ↑↑ |
| ApoA-I | 29.0 | ↓ | ↑ | ↑ |
| ApoE | 33.0 | ↓ | ↑↑ | ↑ |
| Oxysterol binding protein-related protein 2, Hepatic lipase / Triacylglycerol lipase | 55.0 | ↓ | ↓ | ND |
| | | | | |

| **Table 3B** | | | | |
|---|---|---|---|---|
| **Protein** | **Molecular weight (kDa)** | **Treatment** | | |
| | | **Sim** | **JC** | **JCH2** |
| ApoC-I | 6.6 | ↑↑ | ↑ | ↑ |
| ApoC-II/III | 8.8 | ↑ | ↑ | ↑ |
| ApoC-IV, SAA-1 protein | 11.0 | ↑↑ | ↑ | ↑ |
| sPLA₂, FABP (aP2) | 14.0 | ↑↑ | ↑ | ↑ |
| PMKase, ApoM | 21.0 | ↓ | ↓ | ↓ |
| StAR related lipid transfer protein | 23.0 | ↓ | ↓ | ↓ |
| ApoE | 33.0 | ↑ | ↑ | ↑ |

| | | | | |
|---|---|---|---|---|
| ↓: down-regulated, ↑: up-regulated, ND: not detected | | | | |

## Claims

1. An isolated nucleic acid molecule selected from the group consisting of:
(a) nucleic acid molecules consisting of the nucleotide sequence of SEQ ID NO:1;
(b) nucleic acid molecules encoding a peptide, wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13;
(c) nucleic acid molecules consisting of a nucleotide sequence having at least 80% identity with the sequence of SEQ ID NO:1 or a fragment thereof having at least 80% identity with the sequence of SEQ ID NO:1 and which encode a peptide having the function of HMGCoA reductase inhibitor, reducing the accumulation of cholesterol in the cholesterol biosynthesis pathway and/or reducing the level of serum cholesterol.

2. The nucleic acid molecule of claim 1, wherein the nucleic acid molecule (c) has a nucleotide sequence having at least 90% identity with the sequence of SEQ ID NO:1 or a fragment thereof having at least 90% identity with the sequence of SEQ ID NO:1 and which encode a peptide having the function of HMGCoA reductase inhibitor, reducing the accumulation of cholesterol in the cholesterol biosynthesis pathway and/or reducing the level of serum cholesterol.

3. The nucleic acid molecule of claims 1 or 2, wherein the nucleic acid molecule is a single or double strand polynucleotide, oligonucleotide, genomic DNA, cDNA, RNA, or mRNA.

4. An expression vector, comprising at least one of the nucleic acid molecules of any one of claims 1 to 3.

5. The expression vector of claim 4, wherein the expression vector further comprises a regulatory nucleic acid sequence.

6. The expression vector of claims 4 or 5, wherein the regulatory nucleic acid sequence is linked to the nucleic acid molecule encoding the polypeptide.

7. The expression vector of any one of claims 4 to 6, wherein the regulatory nucleic acid is a prokaryotic or eukaryotic promoter.

8. The expression vector of any one of claims 4 to 7, wherein at least two from the nucleic acid molecules of (a), (b) and (c) are fused together in the vector.

9. A host cell, wherein the host cell comprises the vector of any one of claims 4 to 8.

10. The host cell of claim 9, wherein the host cell is in the form of cell culture.

11. The host cell of claims 9 or 10, wherein the host cell is a prokaryotic or eukaryotic cell.

12. The host cell of any one of claims 9 to 11. wherein the host cell is cultured to express a peptide, wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7. SEQ ID NO:8, SEQ ID NO:9, SEQ ID NO:10, SEQ ID NO:11, SEQ ID NO:12 or SEQ ID NO: 13.

13. An isolated peptide, wherein the peptide is a fragment of SEQ ID NO:2 and comprises the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13.

14. The peptide of claim 13, wherein the peptide is a peptide comprising the amino acid sequence of SEQ ID NO:3.

15. The peptide of claim 13 or 14, wherein the peptide is isolated and/or purified from a human or non-human animal species.

16. The peptide of any one of claims 13 to 15, wherein the peptide is isolated and/or purified from the venom.

17. The peptide of claim 16, wherein the venom is from *Buthus martensii* Karsch.

18. The peptide of any one of claims 13 to 17, wherein the peptide is obtained from steps comprising:
- obtaining crude venom;
- carrying out gel filtration; and
- performing reverse-phase high performance liquid chromatography.

19. The peptide of any one of claims 13 to 18, wherein the molecular weight of the peptide is 16803 Da, 16790 Da ; 16791 Da or 17211 Da.

20. A pharmaceutical preparation comprising the peptide of any one of claims 13 to 19.

21. The pharmaceutical preparation of claim 20, wherein the pharmaceutical preparation further comprises a pharmaceutically acceptable carrier, diluent, excipient or a combination thereof.

22. The pharmaceutical preparation of claim 20 or 21, wherein the pharmaceutical preparation is in the form of oral, parenteral, injection, topical, and/or implant preparation.

23. Use of at least one peptide, wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13, for the preparation of a medicament for the treatment or prophylaxis of disorders **characterised by** the accumulation of cholesterol, its by-product and/or related lipid derived products.

24. The use of claim 23, wherein the medicament comprises at least one peptide comprising the amino acid sequence of SEQ ID NO:3.

25. Use of at least one peptide of any one of claims 13 to 19 for the preparation of a medicament for the treatment or prophylaxis of disorders **characterised by** the accumulation of cholesterol, its by-product and/or related lipid derived products.

26. The use of any one of claims 23 to 25, wherein the by-product comprises bile acid.

27. The use of any one of claims 23 to 26, wherein the related lipid derived products comprises HDL, LDL and/or VLDL.

28. The use of any one of claims 23 to 27, wherein the disorders comprise hypertension, atherosclerosis, stroke, neurovascular and/or cardiovascular disorders.

29. The use of any one of claims 23 to 28, wherein the medicament further comprises a pharmaceutically acceptable carrier, diluent, excipient or a combination thereof.

30. The use of any one of claims 23 to 29, wherein the medicament is administered locally, by injection, implantation, topical administration to a tissue locus, parenterally and/or orally.

31. Use of at least one peptide, wherein the peptide is a fragment of SEQ ID NO:2, comprising the amino acid sequence of SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 or SEQ ID NO:13 for the preparation of a medicament for the treatment for cholesterol independent and pleiotropic conditions.

32. The use of claim 31, wherein the medicament comprises at least one peptide comprising the amino acid sequence of SEQ ID NO:3.

33. Use of at least one peptide of any one of claims 13 to 19 for the preparation of a medicament for the treatment for cholesterol independent and pleiotropic conditions.

34. The use of any one of claims 31 to 33, wherein the cholesterol independent and pleiotropic condition comprise atherosclerotic stabilization, amelioration of endothelial dysfunction, improved coronary artery compliance, prevention of plaque rupture, and/or thrombus formation.

## Patentansprüche

1. Isoliertes Nukleinsäuremolekül ausgewählt aus der Gruppe bestehend aus:
(a) Nukleinsäuremolekülen bestehend aus der Nukleotidsequenz von SEQ ID NO: 1;
(b) Nukleinsäuremolekülen, die für ein Peptid codieren, wobei das Peptid ein Fragment von SEQ ID NO: 2 ist, das die Aminosäuresequenz von SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 oder SEQ ID NO: 13 umfasst;
(c) Nukleinsäuremolekülen, die aus einer Nukleotidsequenz bestehen mit einer Identität von wenigstens 80 % bezüglich der Sequenz von SEQ ID NO: 1 oder einem Fragment davon mit einer Identität von wenigstens 80 % bezüglich der Sequenz von SEQ ID NO: 1 und die für ein Peptid codieren, das die Funktion eines HMGCoA-Reduktase-Inhibitors aufweist, das die Akkumulierung von Cholesterol in dem Cholesterol-Biosyntheseweg verringert und/oder den Titer an Serumcholesterol verringert.

2. Nukleinsäuremolekül nach Anspruch 1, wobei das Nukleinsäuremolekül (c) eine Nukleotidsequenz aufweist mit einer Identität von wenigstens 90 % bezüglich der Sequenz von SEQ ID NO: 1 oder ein Fragment davon mit einer Identität von wenigstens 90 % bezüglich der Sequenz von SEQ ID NO: 1 und die/das ein Peptid codiert, das die Funktion eines HMGCoA-Reduktase-Inhibitors aufweist, das die Akkumulierung von Cholesterol in dem Cholesterol-Biosynheseweg verringert und/oder den Titer an Serumcholesterol verringert.

3. Nukleinsäuremolekül nach Anspruch 1 oder 2, wobei das Nukleinsäuremolekül ein einzel- oder doppelsträngiges Polynukleotid, ein Oligonukleotid, eine genomische DNA, eine cDNA, eine RNA oder eine mRNA ist.

4. Expressionsvektor, umfassend wenigstens eines der Nukleinsäuremoleküle nach einem der Ansprüche 1 bis 3.

5. Expressionsvektor nach Anspruch 4, wobei der Expressionsvektor weiter eine regulatorische Nukleinsäuresequenz umfasst.

6. Expressionsvektor nach Anspruch 4 oder 5, wobei die regulatorische Nukleinsäuresequenz mit dem Nukleinsäuremolekül verbunden ist, das für das Polypeptid codiert.

7. Expressionsvektor nach einem der Ansprüche 4 bis 6, wobei die regulatorische Nukleinsäuresequenz ein prokaryotischer oder eukaryotischer Promotor ist.

8. Expressionsvektor nach einem der Ansprüche 4 bis 7, wobei wenigstens zwei der Nukleinsäuremoleküle gemäß (a), (b) und (c) in dem Vektor miteinander fusioniert sind.

9. Wirtszelle, wobei die Wirtszelle den Vektor nach einem der Ansprüche 4 bis 8 umfasst.

10. Wirtszelle nach Anspruch 9, wobei die Wirtszelle in der Form einer Zellkultur vorliegt.

11. Wirtszelle nach Anspruch 9 oder 10, wobei die Wirtszelle eine prokaryotische oder eukaryotische Zelle ist.

12. Wirtszelle nach einem der Ansprüche 9 bis 11, wobei die Wirtszelle kultiviert wird, um ein Peptid zu exprimieren, wobei das Peptid ein Fragment von SEQ ID NO: 2 ist, das die Aminosäuresequenz von SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 oder SEQ ID NO: 13 umfasst.

13. Isoliertes Pepid, wobei das Peptid ein Fragment von SEQ ID NO: 2 ist und die Aminosäuresequenz von SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 oder SEQ ID NO: 13 umfasst.

14. Peptid nach Anspruch 13, wobei das Peptid ein Peptid ist, welches die Aminosäuresequenz von SEQ ID NO: 3 umfasst.

15. Peptid nach Anspruch 13 oder 14, wobei das Peptid isoliert ist und/oder gereinigt ist aus einer menschlichen oder nicht-menschlichen Tierart.

16. Peptid nach einem der Ansprüche 13 bis 15, wobei das Peptid aus dem tierischen Gift isoliert und/oder gereinigt ist.

17. Peptid nach Anspruch 16, wobei das tierische Gift von *Buthus martensii* Karsch ist.

18. Peptid nach einem der Ansprüche 13 bis 17, wobei das Peptid erhalten ist durch Schritte umfassend:
- Erhalten des rohen tierischen Giftes;
- Durchführen einer Gelfiltration; und
- Durchführen einer Umkehrphasen-Hochleistungsflüssigchromatographie.

19. Peptid nach einem der Ansprüche 13 bis 18, wobei das Molekulargewicht des Peptids 16803 Da, 16790 Da, 16791 Da oder 17211 Da beträgt.

20. Pharmazeutisches Präparat umfassend das Peptid nach einem der Ansprüche 13 bis 19.

21. Pharmazeutisches Präparat nach Anspruch 20, wobei das pharmazeutische Präparat weiter einen pharmazeutisch akzeptablen Träger, ein pharmazeutisch akzeptables Verdünnungsmittel, ein pharmazeutisch akzeptables Bindemittel oder eine Kombination davon umfasst.

22. Pharmazeutisches Präparat nach Anspruch 20 oder 21, wobei das pharmazeutische Präparat in der Form eines oralen, parenteralen, Injektions-, topischen und/oder Implantatpräparates vorliegt.

23. Verwendung wenigstens eines Peptids, wobei das Peptid ein Fragment von SEQ ID NO: 2 ist, das die Aminosäuresequenz von SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 oder SEQ ID NO: 13 umfasst, zur Herstellung eines Medikaments für die Behandlung oder Prophylaxe von Erkrankungen, die durch die Akkumulierung von Cholesterol, seinem Nebenprodukt und/oder verwandten Lipid-abgeleiteten Produkten **gekennzeichnet** sind.

24. Verwendung nach Anspruch 23, wobei das Medikament wenigstens ein Peptid umfasst, welches die Aminosäuresequenz von SEQ ID NO: 3 umfasst.

25. Verwendung von wenigstens einem Peptid nach einem der Ansprüche 13 bis 19 für die Herstellung eines Medikaments für die Behandlung oder Prophylaxe von Erkrankungen, die durch die Akkumulierung von Cholesterol, seinem Nebenprodukt und/oder verwandten Lipid-abgeleiteten Produkten **gekennzeichnet** ist.

26. Verwendung nach einem der Ansprüche 23 bis 25, wobei das Nebenprodukt Gallensäure umfasst.

27. Verwendung nach einem der Ansprüche 23 bis 26, wobei die verwandten Lipid-abgeleiteten Produkte HDL, LDL und/oder VLDL umfassen.

28. Verwendung nach einem der Ansprüche 23 bis 27, wobei die Erkrankungen Bluthochdruck, Atherosklerose, Schlaganfall, neurovaskuläre und/oder kardiovaskuläre Erkrankungen umfassen.

29. Verwendung nach einem der Ansprüche 23 bis 28, wobei das Medikament weiter einen pharmazeutisch akzeptablen Träger, ein pharmazeutisch akzeptables Verdünnungsmittel, ein pharmazeutisch akzeptables Bindemittel oder eine Kombination davon umfasst.

30. Verwendung nach einem der Ansprüche 23 bis 29, wobei das Medikament lokal verabreicht wird, durch Injektion, Implantation, topische Verabreichung an einen Gewebelokus, parenteral und/oder oral.

31. Verwendung wenigstens eines Peptids, wobei das Peptid ein Fragment von SEQ ID NO: 2 ist, das die Aminosäuresequenz von SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 oder SEQ ID NO: 13 umfasst, zur Herstellung eines Medikaments für die Behandlung von Cholesterol-unabhängigen und pleiotropen Zuständen.

32. Verwendung nach Anspruch 31, wobei das Medikament wenigstens ein Peptid umfasst, welches die Aminosäuresequenz von SEQ ID NO: 3 umfasst.

33. Verwendung von wenigstens einem Peptid nach einem der Ansprüche 13 bis 19 für die Herstellung eines Medikaments für die Behandlung von Cholesterol-unabhängigen und pleiotropen Zuständen.

34. Verwendung nach einem der Ansprüche 31 bis 33, wobei der Cholesterol-unabhängige und pleiotrope Zustand atherosklerotische Stabilisierung, Verbesserung von Endotheldysfunktion, verbesserte Koronararteriencompliance, Verhinderung von Plaqueruptur und/oder Thrombusbildung umfasst.

## Revendications

1. Molécule d'acide nucléique isolée choisie dans le groupe constitué par :
(a) des molécules d'acide nucléique constituées par la séquence de nucléotides de SEQ ID NO : 1 ;
(b) des molécules d'acide nucléique codant pour un peptide, le peptide étant un fragment de SEQ ID NO : 2, comprenant la séquence d'acides aminés de SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12 ou SEQ ID NO : 13 ;
(c) des molécules d'acide nucléique constituées par une séquence de nucléotides présentant une identité d'au moins 80 % avec la séquence de SEQ ID NO : 1 ou un fragment de celle-ci présentant une identité d'au moins 80 % avec la séquence de SEQ ID NO : 1 et qui codent pour un peptide ayant la fonction d'inhibiteur de l'HMGCoA réductase, réduisant l'accumulation de cholestérol dans la voie de biosynthèse du cholestérol et/ou réduisant le niveau de cholestérol sérique.

2. Molécule d'acide nucléique selon la revendication 1, dans laquelle la molécule d'acide nucléique (c) possède une séquence de nucléotides présentant une identité d'au moins 90 % avec la séquence de SEQ ID NO : 1 ou un fragment de celle-ci présentant une identité d'au moins 90 % avec la séquence de SEQ ID NO : 1 et qui code pour un peptide ayant la fonction d'inhibiteur de l'HMGCoA réductase, réduisant l'accumulation de cholestérol dans la voie de biosynthèse du cholestérol et/ou réduisant le niveau de cholestérol sérique.

3. Molécule d'acide nucléique selon les revendications 1 ou 2, la molécule d'acide nucléique étant un polynucléotide, oligonucléotide, ADN génomique, ADNc, ARN, ou ARNm simple brin ou double brin.

4. Vecteur d'expression comprenant au moins l'une des molécules d'acide nucléique selon l'une quelconque des revendications 1 à 3.

5. Vecteur d'expression selon la revendication 4, le vecteur d'expression comprenant en outre une séquence d'acide nucléique régulatrice.

6. Vecteur d'expression selon les revendications 4 ou 5, dans lequel la séquence d'acide nucléique régulatrice est liée à la molécule d'acide nucléique codant pour le polypeptide.

7. Vecteur d'expression selon l'une quelconque des revendications 4 à 6, dans lequel l'acide nucléique régulateur est un promoteur procaryote ou eucaryote.

8. Vecteur d'expression selon l'une quelconque des revendications 4 à 7, dans lequel au moins deux des molécules d'acide nucléique de (a), (b) et (c) sont fusionnées l'une à l'autre (les unes aux autres) dans le vecteur.

9. Cellule hôte, la cellule hôte comprenant le vecteur selon l'une quelconque des revendications 4 à 8.

10. Cellule hôte selon la revendication 9, la cellule hôte se trouvant sous la forme de culture cellulaire.

11. Cellule hôte selon les revendications 9 ou 10, la cellule hôte étant une cellule procaryote ou eucaryote.

12. Cellule hôte selon l'une quelconque des revendications 9 à 11, la cellule hôte étant cultivée pour exprimer un peptide, le peptide étant un fragment de SEQ ID NO : 2, comprenant la séquence d'acides aminés de SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12 ou SEQ ID NO : 13.

13. Peptide isolé, le peptide étant un fragment de SEQ ID NO : 2 et comprenant la séquence d'acides aminés de SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12 ou SEQ ID NO : 13.

14. Peptide selon la revendication 13, le peptide étant un peptide comprenant la séquence d'acides aminés de SEQ ID NO : 3.

15. Peptide selon la revendication 13 ou 14, le peptide étant isolé et/ou purifié d'une espèce animale humaine ou non humaine.

16. Peptide selon l'une quelconque des revendications 13 à 15, le peptide étant isolé et/ou purifié du venin.

17. Peptide selon la revendication 16, le venin provenant de *Buthus martensii* Karsch.

18. Peptide selon l'une quelconque des revendications 13 à 17, le peptide étant obtenu par les étapes comprenant :
- l'obtention du venin brut ;
- la réalisation d'une filtration sur gel, et
- la réalisation d'une chromatographie liquide haute performance en phase inverse.

19. Peptide selon l'une quelconque des revendications 13 à 18, la masse moléculaire du peptide étant de 16 803 Da, 16 790 Da ; 16 791 Da ou 17 211 Da.

20. Préparation pharmaceutique comprenant le peptide selon l'une quelconque des revendications 13 à 19.

21. Préparation pharmaceutique selon la revendication 20, la préparation pharmaceutique comprenant en outre un véhicule, un diluant ou un excipient pharmaceutiquement acceptables ou une combinaison de ceux-ci.

22. Préparation pharmaceutique selon la revendication 20 ou 21, la préparation pharmaceutique se trouvant sous la forme d'une préparation orale, parentérale, pour injection, topique, et/ou pour implant.

23. Utilisation d'au moins un peptide, le peptide étant un fragment de SEQ ID NO : 2, comprenant la séquence d'acides aminés de SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12 ou SEQ ID NO : 13, pour la préparation d'un médicament destiné au traitement ou à la prophylaxie de troubles **caractérisés par** l'accumulation de cholestérol, de ses produits intermédiaires et/ou de produits apparentés, dérivés de lipides.

24. Utilisation selon la revendication 23, dans laquelle le médicament comprend au moins un peptide comprenant la séquence d'acides aminés de SEQ ID NO : 3.

25. Utilisation d'au moins un peptide selon les revendications 13 à 19, pour la préparation d'un médicament destiné au traitement ou à la prophylaxie de troubles **caractérisés par** l'accumulation de cholestérol, de ses produits intermédiaires et/ou de produits apparentés, dérivés de lipides.

26. Utilisation selon l'une quelconque des revendications 23 à 25, dans laquelle le produit intermédiaire comprend l'acide biliaire.

27. Utilisation selon l'une quelconque des revendications 23 à 26, dans laquelle les produits apparentés dérivés de lipides comprennent le HDL, le LDL et/ou le VLDL.

28. Utilisation selon l'une quelconque des revendications 23 à 27, dans laquelle le trouble comprend l'hypertension, l'athérosclérose, un accident vasculaire cérébral, des troubles neurovasculaires et/ou cardiovasculaires.

29. Utilisation selon l'une quelconque des revendications 23 à 28, dans laquelle le médicament comprend en outre un véhicule, un diluant ou un excipient pharmaceutiquement acceptables ou une combinaison de ceux-ci.

30. Utilisation selon l'une quelconque des revendications 23 à 29, dans laquelle le médicament est administré par voie locale, par injection, par implantation, par administration topique à un site tissulaire, par voie parentérale et/ou orale.

31. Utilisation d'au moins un peptide, le peptide étant un fragment de SEQ ID NO : 2, comprenant la séquence d'acides aminés de SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12 ou SEQ ID NO: 13 pour la préparation d'un médicament destiné au traitement de conditions indépendantes du cholestérol et pléiotropes.

32. Utilisation selon la revendication 31, dans laquelle le médicament comprend au moins un peptide comprenant la séquence d'acides aminés de SEQ ID NO : 3.

33. Utilisation d'au moins un peptide selon les revendications 13 à 19, pour la préparation d'un médicament destiné au traitement de conditions indépendantes du cholestérol et pléiotropes.

34. Utilisation selon l'une quelconque des revendications 31 à 33, dans laquelle la condition indépendante du cholestérol et pléiotrope comprend la stabilisation athérosclérotique, une amélioration d'un dysfonctionnement endothélial, une amélioration de la compliance des artères coronaires, la prévention d'une rupture de plaque, et/ou la formation de thrombus.


### SEQUENCE LISTING

<110> INTRO
<120> A novel HMGCoA reductase inhibitor
<130> FP2262
<160> 18
<170> Patent In version 3.2
<210> 1
   <211> 335
   <212> DNA
   <213> JCH2
<220>
   <221> CDS
   <222> (22)..(303)
<400> 1
   ggtacatttc taaaaaaagt t atg gtg aaa atg caa gtt att ttc att gct       51
                           Met Val Lys Met Gln Val Ile Phe Ile Ala
                           1               5                   10
   ttc atc gct gta ata gca tgt agc atg gta tat gga gat agt ctt tcc       99
   Phe Ile Ala Val Ile Ala Cys Ser Met Val Tyr Gly Asp Ser Leu Ser
                   15                  20                  25
   cct tgg aat gaa ggc gat acg tat tac ggt tgc cag aga caa acg gat      147
   Pro Trp Asn Glu Gly Asp Thr Tyr Tyr Gly Cys Gln Arg Gln Thr Asp
               30                  35                  40
   gaa ttc tgt aat aaa att tgt aag ctg cac tta gca agc ggt gga agc      195
   Glu Phe Cys Asn Lys Ile Cys Lys Leu His Leu Ala Ser Gly Gly Ser
           45                  50                  55
   tgt cag caa ccc gct cct ttt gtg aaa tta tgc aca tgc caa ggt att      243
   Cys Gln Gln Pro Ala Pro Phe Val Lys Leu Cys Thr Cys Gln Gly Ile
       60                  65                  70
   gat tac gac aac agt ttc ttt ttt gga gca ttg gaa aaa caa tgt cct      291
   Asp Tyr Asp Asn Ser Phe Phe Phe Gly Ala Leu Glu Lys Gln Cys Pro
   75                  80                  85                  90
   aaa tta aga gag tagccgaaag atttgcattt atcaatgcta tt                  335
   Lys Leu Arg Glu
<210> 2
   <211> 94
   <212> PRT
   <213> JCH2
<400> 2
   Met Val Lys Met Gln Val Ile Phe Ile Ala Phe Ile Ala Val Ile Ala
   1 5 10 15
   Cys Ser Met Val Tyr Gly Asp Ser Leu Ser Pro Trp Asn Glu Gly Asp
   20 25 30
   Thr Tyr Tyr Gly Cys Gln Arg Gln Thr Asp Glu Phe Cys Asn Lys Ile
   35 40 45
   Cys Lys Leu His Leu Ala Ser Gly Gly Ser Cys Gln Gln Pro Ala Pro
   50 55 60
   Phe Val Lys Leu Cys Thr Cys Gln Gly Ile Asp Tyr Asp Asn Ser Phe
   65 70 75 80
   Phe Phe Gly Ala Leu Glu Lys Gln Cys Pro Lys Leu Arg Glu
   85 90
<210> 3
   <211> 72
   <212> PRT
   <213> Deduced amino acid sequence of JCH2
<400> 3
   Asp Ser Leu Ser Pro Trp Asn Glu Gly Asp Thr Tyr Tyr Gly Cys Gln
   1 5 10 15
   Arg Gln Thr Asp Glu Phe Cys Asn Lys Ile Cys Lys Leu His Leu Ala
   20 25 30
   Ser Gly Gly Ser Cys Gln Gln Pro Ala Pro Phe Val Lys Leu Cys Thr
   35 40 45
   Cys Gln Gly Ile Asp Tyr Asp Asn Ser Phe Phe Phe Gly Ala Leu Glu
   50 55 60
   Lys Gln Cys Pro Lys Leu Arg Glu
   65 70
<210> 4
   <211> 30
   <212> PRT
   <213> N-terminal sequence obtained from amino acid sequence of JCH2
<400> 4
   Asp Ser Leu Ser Pro Trp Asn Glu Gly Asp Thr Tyr Tyr Gly Cys Gln
   1 5 10 15
   Arg Gln Thr Asp Glu Phe Cys Asn Lys Ile Cys Lys Leu His
   20 25 30
<210> 5
   <211> 14
   <212> PRT
   <213> Peptide 1 from RP-HPLC of JCH2 deduced amino acid sequence
<400> 5
   Gln Pro Ala Pro Phe Val Lys Leu Cys Thr Cys Gln Gly Ile
   1 5 10
<210> 6
   <211> 19
   <212> PRT
   <213> Peptide 2 from RP-HPLC of JCH2 deduced amino acid sequence
<400> 6
   Lys Leu His Leu Ala Ser Gly Gly Ser Cys Gln Gln Pro Ala Pro Phe
   1 5 10 15
   Val Lys Leu
<210> 7
   <211> 22
   <212> PRT
   <213> Peptide 3 from RP-HPLC of JCH2 deduced amino acid sequence
<400> 7
   Pro Ala Pro Phe Val Lys Leu Cys Thr Cys Gln Gly Ile Asp Tyr Asp
   1 5 10 15
   Asn Ser Phe Phe Phe Gly
   20
<210> 8
   <211> 20
   <212> PRT
   <213> Peptide 4 from RP-HPLC of JCH2 deduced amino acid sequence
<400> 8
   Gln Gly Ile Asp Tyr Asp Asn Ser Phe Phe Phe Gly Ala Leu Glu Lys
   1 5 10 15
   Gln Cys Pro Lys
   20
<210> 9
   <211> 21
   <212> PRT
   <213> Peptide 5 from RP-HPLC of JCH2 deduced amino acid sequence
<400> 9
   Gly Cys Gln Arg Gln Thr Asp Glu Phe Cys Asn Lys Ile Cys Lys Leu
   1 5 10 15
   His Leu Ala Ser Gly
   20
<210> 10
   <211> 14
   <212> PRT
   <213> Peptide 6 from RP-HPLC of JCH2 deduced amino acid sequence
<400> 10
   Asp Ser Leu Ser Pro Trp Asn Glu Gly Asp Thr Tyr Tyr Gly
   1 5 10
<210> 11
   <211> 15
   <212> PRT
   <213> Peptide 7 from RP-HPLC of JCH2 deduced amino acid sequence
<400> 11
   Leu Ser Pro Trp Asn Glu Gly Asp Thr Tyr Tyr Gly Cys Gln Arg
   1 5 10 15
<210> 12
   <211> 7
   <212> PRT
   <213> Peptide 8 from RP-HPLC of JCH2 deduced amino acid sequence
<400> 12
   Ser Pro Trp Asn Glu Gly Asp
   1 5
<210> 13
   <211> 6
   <212> PRT
   <213> Peptide 9 from RP-HPLC of JCH2 deduced amino acid sequence
<400> 13
   Gly Asp Thr Tyr Tyr Gly
   1 5
<210> 14
   <211> 23
   <212> DNA
   <213> Artificial Sequence
<220>
   <223> Gene specific primer forward 1
<220>
   <221> misc feature
   <222> (3)..(3)
   <223> y is t or c
<220>
   <221> misc_feature
   <222> (6)..(6)
   <223> y is t or c
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> y is t or c
<220>
   <221> misc_feature
   <222> (21)..(21)
   <223> y is t or c
<400> 14
   gayagyctnt cnccytggaa yga 23
<210> 15
   <211> 22
   <212> DNA
   <213> Artificial Sequence
<220>
   <223> Universal adaptor primer as reverse primer, AP1
<400> 15
   gtaatacgac tcactatagg gc 22
<210> 16
   <211> 21
   <212> DNA
   <213> Artificial Sequence
<220>
   <223> Gene specific reverse primer
<400> 16
   attccaaggg gaaagactat c 21
<210> 17
   <211> 24
   <212> DNA
   <213> Artificial Sequence
<220>
   <223> Gene specific forward primer
<400> 17
   ggtacatttc taaaaaaagt tatg 24
<210> 18
   <211> 24
   <212> DNA
   <213> Artificial Sequence
<220>
   <223> Gene specific reverse primer
<400> 18
   aatagcattg attaaatgca aatc 24